# EUROPEAN PATENT SPECIFICATION

(11) **EP 3 007 721 B1**
(45) Date of publication and mention of the grant of the patent: **27.02.2019**
(21) Application number: 14810520.8
(22) Date of filing: 13.06.2014
(51) Int. Cl.: A61K 47/60, A61K 38/22, A61K 38/00, A61K 38/28, A61K 45/06, C07K 14/575

(54) **NON-AGGLOMERATING BIOCONJUGATES OF AMYLIN-MIMETIC COMPOUNDS AND POLYETHYLENEGLYCOL**
NICHTAGGLOMERIERENDE BIOKONJUGATE VON AMYLINMIMETISCHEN VERBINDUNGEN UND POLYETHYLENGLYKOL
BIOCONJUGUÉS DE COMPOSÉS MIMÉTIQUE D'AMYLINE ET DE POLYÉTHYLÈNEGLYCOL NE S'AGGLOMÉRANT PAS

(30) Priority: 14.06.2013 BR 102013017626
(43) Date of publication of application: 20.04.2016
(73) Proprietor: Universidade Federal Do Rio De Janeiro, 21941-901 Rio de Janeiro - RJ (BR)
(72) Inventor: TRAMBAIOLI DA ROCHA E LIMA, Luis Mauricio, CEP 22470-130 Rio de Janeiro - RJ (BR); GUERREIRO ROSADO, Luiz Henrique, CEP 23835-180 Seropédica - RJ (BR); AVILA NETTO GUTERRES, Mariana Fernandes de, CEP 21330-600 Rio de Janeiro - RJ (BR); MELO VIEIRA GONÇALVES FERREIRA, Bruno, CEP 20270-002 Rio de Janeiro - RJ (BR)
(74) Representative: Potter Clarkson
(86) International application number: PCT/BR2014/000199
(87) International publication number: WO 2014/197961

(56) References cited:
- EP-A1- 2 036 923
- WO-A1-2013/009539
- WO-A1-2013/059336
- WO-A2-2007/104789
- BR-A2- PI1 003 424
- US-A1- 2010 121 032
- ANTONINO MAZZAGLIA ET AL: "Aggregation Properties of the Peptide Fragments Derived from the 17-29 Region of the Human and Rat IAPP: A Comparative Study with Two PEG-Conjugated Variants of the Human Sequence", JOURNAL OF PHYSICAL CHEMISTRY PART B: CONDENSED MATTER, MATERIALS, SURFACES, INTERFACES & BIOPHYSICAL, vol. 114, no. 2, 21 January 2010 (2010-01-21), pages 705-713, XP055313388, US ISSN: 1520-6106, DOI: 10.1021/jp908436s
- THÁYNA SISNANDE ET AL: "Monoconjugation of Human Amylin with Methylpolyethyleneglycol", PLOS ONE, vol. 10, no. 10, 8 October 2015 (2015-10-08), page e0138803, XP055313303, DOI: 10.1371/journal.pone.0138803
- GUTERRES M F ET AL.: 'Amylin conjugation with methoxyl polyethyleneglycol' PROTEIN & PEPTIDE LETTERS vol. 20, no. 11, November 2013, pages 1264 - 1271, XP008180630
- GUERREIRO L H ET AL.: 'Preparation and characterization of PEGylated amylin' AMERICAN ASSOCIATION OF PHÁRMACEUTICAL SCIENTISTS. vol. 14, no. 3, September 2013, pages 1083 - 1097, XP055279247
- SUN C ET AL.: 'Bifunctional PEGylated exenatide-amylinomimetic hybrids to treat metabolic disorders: an example of long-acting dual hormonal therapeutics' JOURNAL OF MEDICINAL CHEMISTRY vol. 56, no. 22, November 2013, pages 9328 - 9341, XP055190642

## Description

The present invention generally concerns new non-agglomerating bioconjugates of amylin-mimetic compounds and polyethylene glycol, their use mainly in the treatment of diseases associated with deposition or accumulation of extracellular amyloid, which contributes to the dysfunction or failure of systemic organs such as the pancreas.

A particular embodiment of the invention, without excluding any other, concerns the bioconjugates of natural or synthetic human amylin and polyethylene glycol, and the use thereof in the treatment of diabetes, and the toxic effects caused by build-up of amylin oligomers on the surface of pancreatic beta-cells membrane. Said build-up contributes to with the pathogenesis of diabetes. Preserving those cells, already deteriorated in diabetic patients, is therefore a benefit sought for in the art.

### BACKGROUND

Amylin, an amyloid polypeptide of the Langerhans islets, identified with CAS RN: 106602-62-4, is a hormone of the pancreatic beta-cells, co-secreted with insulin in response to feeding, and complements the effects of insulin on the control of postprandial glucose. It is a 37-aminoacid polypeptide, co-secreted with insulin in a proportion insulin:amylin of 100:1.

The beneficial activities of amylin in diabetes and obesity are known: increase in satiety leading to reduced ingestion of food and consequent body weight reduction; slower gastric emptying, improved glucose metabolism profiles with postprandial peaks and reduction of glucagon levels in diabetic patients.

Type 1 diabetes patients have practically no natural amylin production, while patients with long-standing type 2 diabetes have lower levels than healthy individuals.

Because of its physicochemical properties, the use of structurally unmodified amylin as a pharmaceutical active is not possible. Its limited solubility in aqueous solution led to the development of a more soluble amylin-mimetic compound, the pramlintide, in which three of 37 aminoacids of murin amylin are replaced by proline. There is a corresponding commercial product of pramlintide named Symlin®.

Although the solubility of pramlintide is greater than that of human amylin, pramlintide is more similar to murine amylin than human, and does not have good stability in neutral pH, so the product Symlin® is provided as an acid solution. Murine amylin shows reduced propensity of amyloid agglomeration compared to human amylin, suggesting a general propensity for amylin and amylin-mimetic substances. This product has to be administered as subcutaneous injections right before the meals, aiming to increase postprandial amylin levels. Because of its short half-life from 10 to 15 min, such injections of pramlintide increase the concentration of amylin in the blood stream as a series of peaks, not able to restore the basal levels of amylin during fasting. In the scientific literature, pramlintide has been associated with increased risk of severe insulin-induced hypoglycemia, and other adverse effects such as nausea, vomiting, anorexia and fatigue.

The technical literature does not appear to have focused on a solution for the agglomerating tendency of human amylin, or amylin deposition and its harmful effects on pancreatic beta-cell membranes; and no solution is available.

Without being bound by theory, there are indications that cholesterol in the plasma membrane of beta-cells is the key factor in regulating the so-called polymerization of amylin, and its deposition upon said membranes. Absence or low level of cholesterol in the plasma membrane - which is an ordinary aspect in diabetes - inhibits the internalization of amylin oligomers, and favors the extracellular accumulation, potentiating amylin cytotoxicity in that state, said toxicity resulting apoptosis or destruction of beta-cells. On the other hand, the cellular internalization of monomeric amylin is independent of the cholesterol content of the plasma membrane.

Briefly, a series of studies correlates amylin polymerization and deposition - with corresponding toxicity - on the surface of the plasma membrane of beta-cells with absence or lesser presence of cholesterol and/or with increased severity of type 2 diabetes mellitus.

Up to the present there are no medicaments or pharmaceutical actives known for the control of glycemia that also inhibit the aggregation of human amylin or amylin-mimetic compounds.

The present invention generally aims at the conjugation of human amylin and polyethylene glycol, and the use thereof towards the inhibition of the extracellular aggregation of amylin, without inducing the toxicity related to the formation of amylin oligomers and clusters.

The text that follows is to be understood with the following aspects: (1) mention of bioconjugates of amylin with polyethylene glycol also encompasses bioconjugates of amylin-mimetic compounds with polyethylene glycol; (2) the expression "amylin-mimetic compounds" also includes, for ease of expression, human amylin, natural or synthetic, (3) mention of diabetes also encompasses other diseases and dysfunctions directly or indirectly related to the agglomeration/deposition of amylin-mimetic compounds.

The use of the bioconjugates of the invention, particularly bioconjugates of human amylin, shows various benefits, particularly higher stability of the diabetic organism, that are highly recommended by physicians as desirable characteristics for medicaments of this nature:
- increase of the plasma half-life, compared with amylin and amylinomimetic substances such as pramlintide, therefore allowing longer permanence in the blood stream;
- lower doses or fewer injections to mimic the effect of natural amylin;
- good control of glycemia;
- reduction of nausea;
- high satiety concerned with feeding;
- reduced speed of gastric emptying;
- higher solubility than human amylin;
- reduction of glucagon levels, therefore improving glucose control.

### DESCRIPTION OF DRAWINGS

- Figure 1 - chromatogram for monopegylated amylin obtained by the reaction of human amylin and mPEG;
- Figure 2 - Mass spectrometry showing characterization of monopegylated human amylin;
- Figures 3 and 4 - assays for RAMP2 and RAMP3 coreceptor binding interaction for free and purified monopegylated human amylin;
- Figure 5 - assay for amyloid aggregates;
- Figure 6 - plasma stability comparison between free amylin and monopegylated human amylin

### DESCRIPTION OF THE INVENTION

The invention generally concerns chemical entities that aim to avoid the typical toxicity caused by human amylin and amylin-mimetic compounds cause, by decreasing or avoiding agglomeration (also mentioned in the literature as polymerization), deposition and fibrillation upon the pancreatic beta-cells and, in consequence, avoiding harmful effects that apoptosis or destruction of said pancreatic beta-cells cause to the human organism.

In the first aspect, the invention concerns new non-agglomerating bioconjugates of human amylin compounds and polyethylene glycol, characterized by the fact that said bioconjugate contains at least one polyethylene glycol unit (therefore monoconjugate or polyconjugate compounds may be obtained), covalently bonded to the two nitrogen atoms originated from the alpha and epsilon amine moieties (lateral chain) of the lysine 1 residue of the amylin polypeptidic chain, wherein the non-agglomerating bioconjugates of human amylin and polyethylene glycol compounds of formula I

(R1-COX)m-R2

where
R1 represents methoxypolyethylene glycol (mPEG) moiety and functional spacers with various average molar masses,
R2 represents human amylin compounds,
X represents NH or O,
m represents the number of units of the mPEG polymer (R1) conjugated to human amylin compounds (R2) obtained from the conjugation of mPEG- succinimidyl with human amylin compounds through an amide or ester bond by reaction of primary amine or hydroxyl functional moieties;
and compounds of formula II

(R1-X)m-R2

where
R1 represents methoxypolyethylene glycol (mPEG) moiety and functional spacers with various average molar masses,
R2 represents human amylin compounds,
X represents NH or O,
m represents the number of units of the mPEG polymer (R1) conjugated to human amylin compounds (R2) obtained from the conjugation of mPEG- aldehyde with human amylin compounds.

Mention of amylin-mimetic compounds in the bioconjugates of the invention encompasses active derivatives of said amylin-mimetic compounds such as salts, isomers, hydrates, solvates, prodrugs, metabolites, polymorphs an isosteres.

It is important to remember that mention of amylin-mimetic compounds, in this text, encompasses human amylin itself, either natural, synthetic or a bio-semi-synthetic.

In another aspect, the present invention concerns the use of the new non-agglomerating bioconjugates of amylin-mimetic compounds with polyethylene glycol, in the prevention of treatment of diseases or problems, caused or favored by the amyloid deposition or accumulation that leads to dysfunction or failure of systemic organs (i.e. organs or tissues external to the central nervous system), such as hyperglycemia, diabetes, low tolerance to glucose or deficient glucose metabolism, obesity, metabolic syndrome and feeding disorders, and indirectly to problems and vascular diseases resulting from increase in blood pressure, such as
atherosclerosis, myocardial infarction, stroke, coronary heart disease, cardiac diseases in general, Alzheimer disease.

In another aspect, the invention concerns the use of the new non-agglomerating bioconjugates of amylin-mimetic compounds with polyethylene glycol for the preparation of low toxicity products, medicaments, compositions and associations, useful in the prevention or treatment of diseases caused or favored by amyloid deposition or accumulation which leads to dysfunction or failure of systemic organs.

Still in another aspect, the invention concerns low toxicity pharmaceutical compositions comprising a therapeutically effective amount of one or more of the new non-agglomerating bioconjugates of amylin-mimetic compounds with polyethylene glycol and one or more pharmaceutically acceptable excipients. Such compositions are adequate for all variety of administration forms such as oral, enteral, parenteral, lingual, sublingual, nasal, dermal, epidermal, transdermal, mucosal, vaginal, rectal, ocular, etc.

The compositions of the invention present themselves in any necessary or adequate dosage forms, such as solutions, suspensions, emulsions, microemulsions, foams, pastes, creams, tablets, capsules (hard or soft, suppositories), bolus, gels, powders, aerosols, sprays, etc.

The pharmaceutically acceptable excipients employed in the compositions of the inventions are known to the person skilled in the art, such as the ones described, for instance, in *"*Remington's Pharmaceutical Sciences", 15th edition, Mack Publishing Co., New Jersey (1991). As known, specific excipients are chosen according to the desired administration route, within the practice of the pharmacological area.

The pharmaceutical compositions of the invention may additionally comprise one or more active principles, distinct from human amylin, such as (without excluding any other) insulin, ions (such as zinc and sodium), antidiabetics, antibiotics, anti-hypertensives, antiretrovirals, etc. Such compositions may be of immediate, retarded or slow release, also including the possibility that the administration of the new bioconjugate of human amylin be concomitant or sequential to other active principles.

Still another aspect of the invention concerns the use of non-agglomerating bioconjugates of human amylin and polyethyleneglycol as an adjuvant in the prevention or treatment of diseases caused or favored by amyloid deposition or accumulation that leads to dysfunction or failure of systemic organs.

Still another aspect of the invention concerns a medicament characterized by the fact that it comprises a therapeutically effective amount of one or more bioconjugates of human amylin and polyethylene glycol.

Still another aspect of the invention concerns bioconjugates of human amylin and polyethylene glycol, as well as products, medicaments, compositions and associations that comprise them, characterized for the use in medical therapy.

Still another aspect of the invention concerns a method or treatment or prevention of diseases caused or favored by amyloid deposition or accumulation, characterized by comprising the administration to a patient of a therapeutically effective amount of one or more bioconjugates of amylin-mimetic compounds, particularly human amylin, and polyethylene glycol.

### EXAMPLES

The examples that follow concern particular embodiments of the present invention and are not intended to, in any way, limit the scope of the attached claims.

### 1. Preparation of a conjugate according to the invention

Reaction for 2h at 25 °C of a 5 mg/mL human amylin solution in the presence of 10 mM PBS (phosphate buffer solution) pH 7.4, and a molar excess of 5 mPEG / 1 human amylin.

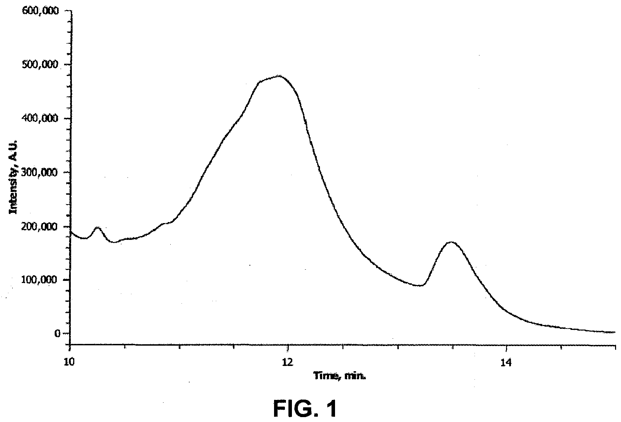

The reaction is quenched by the addition of an equal amount of 30 % acetonitrile/0.1 % trifluoroacetic acid (in water) and then chromatographed in a C18 Kromasil reversed phase column at 4 mL/min, detector set at 220 nm. (Kromasil is a product line commercialized by Separation Products group, a department of AkzoNobel company, Sweden). This purification process is shown in figure 1. The chromatogram shows a peak of monoPEGylated amylin at 12 minutes.

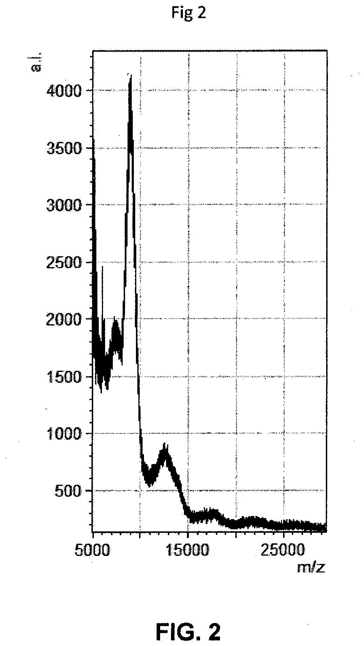

Matrix-assisted laser desorption and ionization-time-of flight mass spectrometry (MALDI-TOF-MS) was performed to characterized monoPEGylated human amylin. Figure 2 shows the peak of monoPEGylated human amylin at 9 Kda.

### 2. Receptor binding assay of human amylin and monoPEGylated human amylin

Purified monoPEGylated human amylin was assayed for RAMP2 and RAMP3 coreceptor binding interaction.

RAMP (receptor activity modifying protein) was labeled with fluorescein isothiocianate for 1 h at 4 °C in PBS (fetal bovine serum) pH 7.4, and purified by size exclusion chromatography (SEC) in Sepharose G25 (agarose-based chromatography media, provided by the US company GE HealthCare) using the same buffer. Labelling was confirmed by UV absorbance measurements at A280 and A490, allowing estimation of coupling efficiency, relying on about 0.5 fluorescein/RAMP molecule. Binding was performed by measuring the fluorescence anisotropy of RAMP-FITC (fluorescein isothiocyanate) as a function of free murine amylin, human amylin-PEGylated and by using the unrelated protein hen egg white lisozyme (HEWL) as a control for non-specific binding.

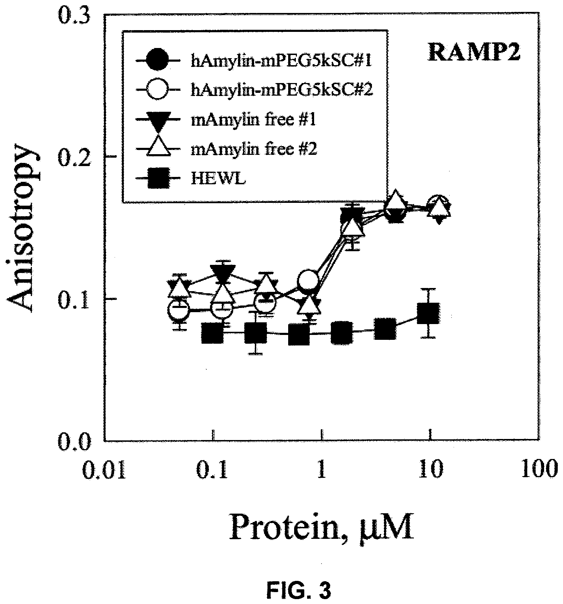

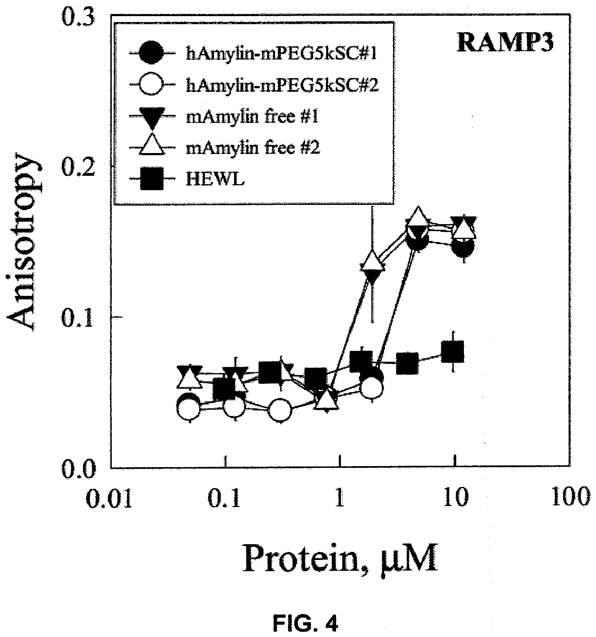

According to Figures 3 and 4, binding assay show a similar apparent binding affinity of both free and purified monoPEGylated human amylin for the coreceptors RAMP2 and RAMP3, in duplicate assays (monoPEGylated human amylin C#1,C#2; and free amylin #1 and #2), indicating that the PEG moiety does not interfere with the coreceptor affinity. Control assay using HEWL show non-specific binding to the coreceptor.

### 3 - Effect of PEGylation on the aggregation profile of human amylin.

Purified monoPEGylated human amylin of example 1 was re-suspended in DMSO and diluted to PBS pH 7.4 and allowed to incubate at 37°C. Samples were evaluated for amyloid aggregates by using the amyloid chromogenic probe thioflavin T (ThT).

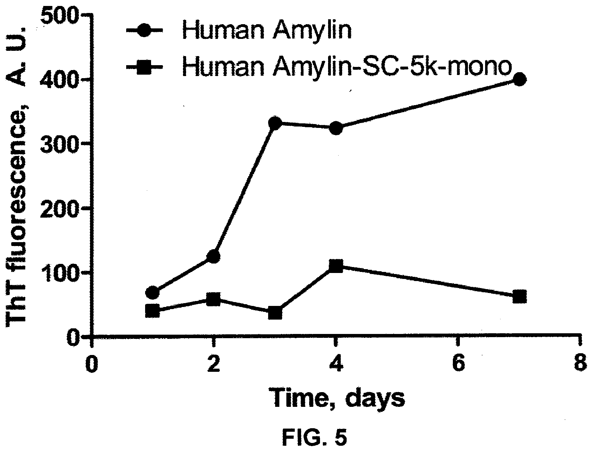

Fig. 5 shows the results. Free Human amylin aggregates very fast. In 3 days, almost all free human amylin was aggregated in amyloid form. MonoPEGylated human amylin, at day 7, still shows no signs of aggregation. This assay proves that the PEGylation process was able to inhibit the agglomeration of human amylin.

### 4 - Pharmacokinetics of the PEGylated human amylin product

The pharmacokinetics of the monoPEGylated human amylin product were characterized in vivo in swiss mice. Mice were housed in a temperature-controlled room with a light-dark cycle of 12 h. Water and food were available ad libitum. Two groups were formed, control (free human amylin, not PEGylated) and PEGylated amylin. Animals received 100 uL of saline containing 10 µg of human amylin peptides, either free human amylin or purified monoPEGylated human amylin. Blood was collected retro-orbitally from mice (n=3 mice for each time interval), and the plasma was subjected to ELISA assay for human amylin as provided by the manufacturer (Millipore, Cat Number EZHA-52K - http://www.millipore.com/catalogue/item/ezha-52k).

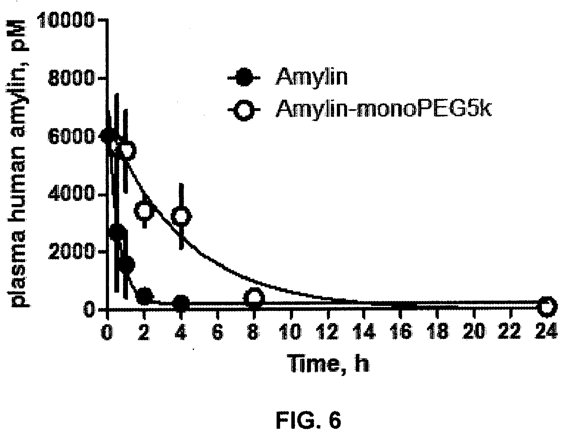

Figure 6 shows fast plasma decay of free amylin, while the monoPEGylated human amylin shows longer duration of stability in plasma. This assay proves that the process of PEGylation of human amylin is capable of increasing its half-life.

## Claims

1. Non-agglomerating bioconjugates of human amylin and polyethylene glycol, wherein said bioconjugate contains at least one polyethylene glycol unit covalently bonded to the nitrogen atom forming the alpha and/or epsilon moieties (lateral chain) of the lysine 1 residue of the amylin polypetidic chain; wherein, the non-agglomerating bioconjugates of human amylin compounds and polyethylene glycol, has the formula I
(R1-COX)m-R2
where R1 represents methoxypolyethylene glycol (mPEG) moiety and functional spacers with various average molar masses,
R2 represents human amylin,
X represents NH or O,
m represents the number of units of the mPEG polymer (R1) conjugated to human amylin (R2) obtained from the conjugation of mPEG- succinimidyl with human amylin, through an amide or ester bond by reaction of primary amine or hydroxyl functional moieties;
or compounds of formula II
(R1X)m-R2
where
R1 represents methoxypolyethylene glycol (mPEG) moiety and functional spacers with various average molar masses,
R2 represents human amylin,
X represents NH or O,
m represents the number of units of the mPEG polymer (R1) conjugated to human amylin (R2) obtained from the conjugation of mPEG- aldehyde with human amylin.

2. Non-agglomerating bioconjugates of human amylin and polyethylene glycol according to claim 1 wherein said human amylin is natural, synthetic or bio-semisynthetic.

3. Non-agglomerating bioconjugates of human amylin and polyethylene glycol according to claim 1 wherein said human amylin is in the form of a salt, isomer, hydrate, solvate, prodrug, polymorph or isostere thereof.

4. Non-agglomerating bioconjugates of human amylin and polyethylene glycol according to claim 1 wherein said bioconjugates, as well as pharmaceutical products, medicaments, compositions and associations comprising said bioconjugates are for use in medical therapy.

5. Non-agglomerating bioconjugates of human amylin and polyethylene glycol according to claim 1 for use in the prevention or treatment of diseases and dysfunction caused or favored by amyloid deposition or accumulation.

6. Non-agglomerating bioconjugates of human amylin and polyethylene glycol for use in claim 5 wherein said diseases or dysfunctions are selected from the group consisting of hyperglycemia, diabetes, low tolerance to glucose or deficient glucose metabolism, obesity, metabolic syndrome, feeding disorders, atherosclerosis, myocardial infarction, stroke, heart coronary disease, heart diseases, and Alzheimer disease; preferably wherein the disease or dysfunction is diabetes mellitus.

7. Non-agglomerating bioconjugates of human amylin and polyethylene glycol according to claim 1 for use in the preparation of low toxicity products useful in the treatment or prevention of diseases caused or favored by amyloid deposition or accumulation.

8. A pharmaceutical composition comprising a therapeutically effective amount of a non-agglomerating bioconjugate of human amylin with polyethylene glycol according to claim 1 and one or more pharmaceutically acceptable excipients.

9. A composition according to claim 8 additionally comprising one or more active ingredients distinct from human amylin.

10. A composition according to claim 9 wherein the said one or more active ingredients distinct from human amylin are selected from the group consisting of insulin, ions such as zinc or sodium, antidiabetics, antibiotics, anti-hypertensives, and antiretrovirals; preferably wherein the one or more active ingredient distinct from human amylin is insulin.

11. An adjuvant comprising a non-agglomerating bioconjugate of a human amylin and polyethylene glycol as described in claim 1 for use in the prevention or treatment of diseases.

12. A medicament comprising a therapeutically effective amount of a non-agglomerating bioconjugate of human amylin and polyethylene glycol as described in claim 1.

13. Process to obtain a bioconjugate of human amylin and PEG comprising the steps of:
a. Reaction of human amylin solution with excess mPEG (methoxypolyethylene glycol);
b. interruption of the reaction;
c. purification of the reaction product;
d. drying of the purified product.

14. Process according to claim 13 wherein
Step a comprises reaction for 2h at 25 °C of a 5 mg/ml human amylin solution in the presence of 10 mM PBS (phosphate buffer solution) pH 7.4, and a molar excess of 5 mPEG / 1 human amylin;
Step b comprises addition of an equal amount of 30 % acetonitrile / 0.1% trifluoroacetic acid in water to quench the reaction.

## Patentansprüche

1. Nicht agglomerierende Biokonjugate von humanem Amylin und Polyethylenglycol, wobei das Biokonjugat mindestens eine Polyethylenglycoleinheit enthält, die kovalent an das die Alpha- und/oder Epsilon-Gruppierungen (seitliche Kette) des Lysin 1-Rests der Amylin-Polypetidkette bildende Stickstoffatom gebunden ist; wobei die nicht agglomerierenden Biokonjugate von humanen Amylinverbindungen und Polyethylenglycol die Formel I haben:
(R1-COX)m-R2
wobei R1 eine Methoxypolyethylenglycol (mPEG)-Gruppierung und funktionelle Spacer mit diversen durchschnittlichen Molmassen darstellt,
R2 humanes Amylin darstellt,
X NH oder O darstellt,
m die Anzahl von Einheiten des an humanes Amylin (R2) konjugierten mPEG-Polymers (R1) darstellt, erhalten aus der Konjugation von mPEG-Succinimidyl mit humanem Amylin, über eine Amid- oder Esterbindung durch die Reaktion von primärem Amin oder funktionellen Hydroxyl-Gruppierungen;
oder Verbindungen der Formel II
(R1X)m-R2
wobei
wobei R1 die Methoxypolyethylenglycol (mPEG)-Gruppierung und funktionelle Spacer mit diversen durchschnittlichen Molmassen darstellt,
R2 humanes Amylin darstellt,
X NH oder O darstellt,
m die Anzahl von Einheiten des an humanes Amylin (R2) konjugierten mPEG-Polymers (R1) darstellt, erhalten aus der Konjugation von mPEG-Aldehyd mit humanem Amylin.

2. Nicht agglomerierende Biokonjugate von humanem Amylin und Polyethylenglycol nach Anspruch 1, wobei das humane Amylin natürlich, synthetisch oder bio-semisynthetisch ist.

3. Nicht agglomerierende Biokonjugate von humanem Amylin und Polyethylenglycol nach Anspruch 1, wobei das humane Amylin in der Form eines Salzes, Isomers, Hydrats, Solvats, Prodrugs, Polymorphs oder Isosters davon vorliegt.

4. Nicht agglomerierende Biokonjugate von humanem Amylin und Polyethylenglycol nach Anspruch 1, wobei die Biokonjugate ebenso wie pharmazeutische Produkte, Medikamente, Zusammensetzungen und Zusammenfügungen, die die Biokonjugate umfassen, zur Verwendung in einer medizinischen Therapie vorgesehen sind.

5. Nicht agglomerierende Biokonjugate von humanem Amylin und Polyethylenglycol nach Anspruch 1 zur Verwendung bei der Prävention oder Behandlung von Krankheiten und Dysfunktionen, die durch Amyloid-Ablagerung oder -Ansammlung bewirkt oder gefördert werden.

6. Nicht agglomerierende Biokonjugate von humanem Amylin und Polyethylenglycol zur Verwendung nach Anspruch 5, wobei die Krankheiten oder Dysfunktionen ausgewählt sind aus der Gruppe bestehend aus Hyperglykämie, Diabetes, geringer Verträglichkeit von Glucose oder unzureichendem Glucosemetabolismus, Adipositas, metabolischem Syndrom, Gefühlsstörungen, Atherosklerose, Myokardinfarkt, Schlaganfall, koronarer Herzkrankheit, Herzkrankheiten und Alzheimer-Erkrankung; wobei bevorzugt die Krankheit oder Dysfunktion Diabetes mellitus ist.

7. Nicht agglomerierende Biokonjugate von humanem Amylin und Polyethylenglycol nach Anspruch 1 zur Verwendung bei der Herstellung von Produkten mit geringer Toxizität, die bei der Behandlung oder Prävention von durch Amyloid-Ablagerung oder -Ansammlung bewirkten oder geförderten Krankheiten einsetzbar sind.

8. Pharmazeutische Zusammensetzung, umfassend eine therapeutisch wirksame Menge eines nicht agglomerierenden Biokonjugats von humanem Amylin mit Polyethylenglycol nach Anspruch 1 und einen oder mehrere pharmazeutisch verträgliche Exzipienten.

9. Zusammensetzung nach Anspruch 8, weiterhin umfassend einen oder mehrere von humanem Amylin verschiedene Wirkstoffe.

10. Zusammensetzung nach Anspruch 9, wobei der eine oder die mehreren von humanem Amylin verschiedenen Wirkstoffe ausgewählt sind aus der Gruppe bestehend aus Insulin, Ionen wie z. B. Zink oder Natrium, Antidiabetika, Antibiotika, Antihypertensiva und antiretroviralen Mitteln; wobei bevorzugt der eine oder die mehreren von humanem Amylin verschiedenen Wirkstoffe Insulin ist.

11. Adjuvans, umfassend ein nicht agglomerierendes Biokonjugat von humanem Amylins und Polyethylenglycol nach Anspruch 1 zur Verwendung bei der Prävention oder Behandlung von Krankheiten.

12. Medikament, umfassend eine therapeutisch wirksame Menge eines nicht agglomerierenden Biokonjugats von humanem Amylin und Polyethylenglycol nach Anspruch 1.

13. Verfahren zum Erhalten eines Biokonjugats von humanem Amylin und PEG, umfassend die folgenden Schritte:
a. Reagieren einer Lösung von humanem Amylin mit einem Überschuss von mPEG (Methoxypolyethylenglycol);
b. Unterbrechen der Reaktion;
c. Reinigen des Reaktionsprodukts;
d. Trocknen des gereinigten Produkts.

14. Verfahren nach Anspruch 13, wobei
Schritt a eine Reaktion einer 5 mg/ml Lösung von humanem Amylin über 2 h bei 25 °C in Gegenwart von 10 mM PBS (Phosphat-Pufferlösung) mit einem pH-Wert von 7,4 und einem molaren Überschuss von 5 mPEG / 1 humanes Amylin umfasst;
Schritt b den Zusatz einer gleichen Menge von 30 % Acetonitril / 0,1 % Trifluoressigsäure in Wasser zum Beenden der Reaktion umfasst.

## Revendications

1. Bioconjugués non agglomérants d'amyline humaine et de polyéthylèneglycol, ledit bioconjugué contenant au moins une unité de polyéthylèneglycol liée de manière covalente à l'atome d'azote formant les fragments alpha et/ou epsilon (chaîne latérale) du résidu de lysine 1 de la chaîne polypeptidique d'amyline ; les bioconjugués non agglomérants de composés d'amyline humaine et de polyéthylèneglycol présentant la formule I
(R1-COX)m-R2
dans laquelle R1 représente un fragment de méthoxypolyéthylène glycol (mPEG) et des espaceurs fonctionnels ayant diverses masses molaires moyennes,
R2 représente de l'amyline humaine,
X représente NH ou O,
m représente le nombre d'unités du polymère mPEG (R1) conjugué à l'amyline humaine (R2) obtenue à partir de la conjugaison de mPEG-succinimidyl avec de l'amyline humaine, par le biais d'une liaison amide ou ester en faisant réagir des fragments fonctionnels d'amine ou d'hydroxyle primaires ;
ou des composés de la formule II
(R1X)m-R2
dans laquelle
R1 représente un fragment de méthoxypolyéthylène glycol (mPEG) et des espaceurs fonctionnels ayant diverses masses molaires moyennes,
R2 représente de l'amyline humaine,
X représente NH ou O,
m représente le nombre d'unités du polymère mPEG (R1) conjugué à l'amyline humaine (R2) obtenue à partir de la conjugaison de mPEG-aldéhyde avec de l'amyline humaine.

2. Bioconjugués non agglomérants d'amyline humaine et de polyéthylèneglycol selon la revendication 1 dans lesquels ladite amyline humaine est naturelle, synthétique ou bio-semi-synthétique.

3. Bioconjugués non agglomérants d'amyline humaine et de polyéthylèneglycol selon la revendication 1 dans lesquels ladite amyline humaine se présente sous la forme d'un sel, d'un isomère, d'un hydrate, d'un solvate, d'un promédicament, d'un polymorphe ou d'un isostère de celle-ci.

4. Bioconjugués non agglomérants d'amyline humaine et de polyéthylèneglycol selon la revendication 1, lesdits bioconjugués, ainsi que les produits pharmaceutiques, les médicaments, les compositions et les associations comprenant lesdits bioconjugués, étant destinés à être utilisés en thérapie médicale.

5. Bioconjugués non agglomérants d'amyline humaine et de polyéthylèneglycol selon la revendication 1 destinés à être utilisés dans la prévention ou le traitement de maladies et de dysfonctionnements dus ou favorisés par le dépôt ou l'accumulation d'amyloïde.

6. Bioconjugués non agglomérants d'amyline humaine et de polyéthylèneglycol selon la revendication 5, lesdits dysfonctionnements ou maladies étant choisis dans le groupe constitué de l'hyperglycémie, du diabète, de la faible tolérance au glucose ou du métabolisme déficient du glucose, de l'obésité, du syndrome métabolique, de troubles de l'alimentation, de l'athérosclérose, de l'infarctus du myocarde, de l'accident vasculaire cérébral, de la maladie cardiaque coronaire, des maladies cardiaques et de la maladie d'Alzheimer ; la maladie ou le dysfonctionnement étant de préférence le diabète sucré.

7. Bioconjugués non agglomérants d'amyline humaine et de polyéthylèneglycol selon la revendication 1 destinés à être utilisés dans la préparation de produits peu toxiques utiles dans le traitement ou la prévention de maladies dues ou favorisées par le dépôt ou l'accumulation d'amyloïde.

8. Composition pharmaceutique comprenant une quantité thérapeutiquement efficace d'un bioconjugué non agglomérant d'amyline humaine et de polyéthylèneglycol selon la revendication 1 et un ou plusieurs excipients pharmaceutiquement acceptables.

9. Composition selon la revendication 8 comprenant en outre un ou plusieurs principes actifs différents de l'amyline humaine.

10. Composition selon la revendication 9 dans laquelle ledit ou lesdits principes actifs différents de l'amyline humaine sont choisis dans le groupe constitué d'insuline, d'ions tels que le zinc ou le sodium, d'antidiabétiques, d'antibiotiques, d'antihypertenseurs et d'antirétroviraux ; le ou les principes actifs différents de l'amyline humaine étant de préférence l'insuline.

11. Adjuvant comprenant un bioconjugué non agglomérant d'une amyline humaine et de polyéthylèneglycol selon la revendication 1 destiné à être utilisé dans la prévention ou le traitement de maladies.

12. Médicament comprenant une quantité thérapeutiquement efficace d'un bioconjugué non agglomérant d'amyline humaine et de polyéthylèneglycol selon la revendication 1.

13. Procédé destiné à obtenir un bioconjugué d'amyline humaine et de PEG comprenant les étapes suivantes :
a. la réaction d'une solution d'amyline humaine avec du mPEG (méthoxypolyéthylène glycol) en excès ;
b. l'interruption de la réaction ;
c. la purification du produit de réaction ;
d. le séchage du produit purifié.

14. Procédé selon la revendication 13 dans lequel
l'étape a comprend la réaction pendant 2 heures à 25°C d'une solution de 5 mg/ml d'amyline humaine en présence de 10 mM de PBS (solution tampon phosphate) à pH 7,4 et d'un excès molaire de 5 mPEG / 1 amyline humaine ;
l'étape b comprend l'ajout d'une quantité égale de 30 % d'acétonitrile / 0,1 % d'acide trifluoroacétique dans de l'eau pour désactiver la réaction.
